(19) Europäisches Patentamt
European Patent Office
Office européen des brevets

(11) Publication number: **0 083 378 A1**

# EUROPEAN PATENT APPLICATION

(21) Application number: 81110691.3

(22) Date of filing: 22.12.81

(51) Int. Cl.³: **C 07 D 211/78**, C 07 D 401/12, A 61 K 31/455

(43) Date of publication of application: 13.07.83 Bulletin 83/28

(84) Designated Contracting States: AT BE CH DE FR GB IT LI NL SE

(71) Applicant: Chugai Seiyaku Kabushiki Kaisha, 5-1, 5-chome, Ukima Kita-ku, Tokyo (JP)

(72) Inventor: Takaku, Sakae, 381-12, Oaza Koizumi, Ageo-shi Saitama-ken (JP)
Inventor: Matsuura, Fumiaki, 3-18-13-1010, Ohgi, Adachi-ku Tokyo (JP)
Inventor: Mori, Takashi, 383, Ohizumigakuenmachi, Nerima-ku Tokyo (JP)
Inventor: Murakami, Yasushi, 2-12-6, Shoan, Suginami-ku Tokyo (JP)
Inventor: Kataoka, Shigeyuki, 2-3-46, Yahata, Sakado-shi Saitama-ken (JP)
Inventor: Takeda, Yasuhisa, Hightown Shiohama 9-809 4-2, Shiohama, Ichikawa-shi Chiba-ken (JP)

(72) Inventor: Yamashita, Yasuhiro, 6-23-10, Togoshi, Shinagawa-ku Tokyo (JP)
Inventor: Takeda, Yumiko, Hightown Shiohama 9-809 4-2, Shiohama, Ichikawa-shi Chiba-ken (JP)
Inventor: Matsuno, Takashi, 813-17, Ohazasashiohgi, Ohmiya-shi Saitama-ken (JP)
Inventor: Mizuno, Koji, 50-8, Kujiraishinden, Kawagoe-shi Saitama-ken (JP)
Inventor: Kaiho, Shinichi, 551, Takashina-cho, Chiba-shi Chiba-ken (JP)
Inventor: Yamazaki, Tamotsu, 732-3, Yamaguchi, Tokorozawa-shi Saitama-ken (JP)
Inventor: Hata, Shun-ichi, 675-14, Nishitani-cho Hodogaya-ku, Yokohama-shi Kanagawa-ken (JP)
Inventor: Takanashi, Shigeru, 277-72, Mizonuma, Asaka-shi Saitama-ken (JP)

(74) Representative: Vossius Vossius Tauchner Heunemann Rauh, Siebertstrasse 4 P.O. Box 86 07 67, D-8000 München 86 (DE)

(54) **Tetrahydronicotinamide derivatives, a process for producing the same and a pharmaceutical composition comprising the same.**

(57) 1,4,5,6-Tetrahydronicotinamide derivatives represented by the formula:

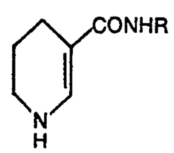

wherein R is a phenyl or pyridyl group which may have one or more substituents, or a salt thereof.

A process of preparing the same and a pharmaceutical composition containing the same.

The compounds suppress the aggregation of platelets, and have antiinflammatory, antipyretic and analgesic activity.

EP 0 083 378 A1

VOSSIUS · VOSSIUS · TAUCHNER · HEUNEMANN · RAUH
PATENTANWÄLTE

SIEBERTSTRASSE 4 · 8000 MÜNCHEN 86 · PHONE: (089) 47 40 75
CABLE: BENZOLPATENT MÜNCHEN · TELEX 5-29 453 VOPAT D

Our Ref.: R 600EP
Case: FP(EPC)/C-1-620

CHUGAI SEIYAKU KABUSHIKI KAISHA
Tokyo / Japan

---

# TETRAHYDRONICOTINAMIDE DERIVATIVES, A PROCESS FOR PRODUCING THE SAME AND A PHARMACEUTICAL COMPOSITION COMPRISING THE SAME

---

This invention relates to 1,4,5,6-tetrahydronicotinamide derivatives of the formula:

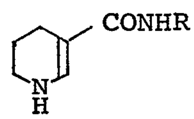

(I)

(wherein R is a phenyl or pyridyl group which may have one or more substituents), a process for producing the same and a pharmaceutical composition comprising the same.

In the formula (I), the substituent of the phenyl and pyridyl is, for example, an alkyl group containing 1-4 carbon atoms, an alkoxy group containing 1-4 carbon atoms or a halogen atom, i.e. a fluorine, chlorine, bromine or iodine atom.

The compounds of this invention are produced by partially reducing, for example, a nicotinamide derivative of the formula:

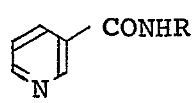

(II)

(wherein R has the same meaning as defined above). The reduction is performed by hydrogenation in the presence of a catalyst and a solvent such as tetrahydrofuran, dioxane, alcohol, hydrous alcohol or acetic acid, preferably alcoholic solvents. Commonly employed metal catalysts are used, and palladium-on-carbon is particularly preferred. A Raney-nickel catalyst may also be used. The reaction temperature is in the range of from 0 to 100°C, preferably

from 10 to 50°C, and the reaction is completed by having the nicotinamide derivative absorb about 2 mols of hydrogen at a hydrogen pressure of 1 to 50 atm., preferably 2 to 5 atm.

The compounds of this invention are useful pharmaceuticals since they have not only a high ability to suppress aggregation of platelets but also antiinflammatory, antipyretic and analgesic activity.

Experiments with animals have shown that many antiinflammatory agents have ability to suppress aggregation of platelets. But it has been pointed out that one defect of these antiinflammatory agents is that when administered to living organisms, they suppress the prostaglandin $I_2$ activity in the walls of the arteries and with extended administration, they accelerate the aggregation of platelets on the arterial walls. The compounds of this invention are free from these defects and are used to prevent and cure thrombosis and are also effective as antiinflammatory and antipyretic analgesic agents.

When used as a medicine, the compounds of this invention are formulated by a conventional technique into tablets, granules, powders, capsules or injection preparations, and administered to patients either orally or parenterally. A tablet, granule, powder or capsule is prepared by mixing the compound with a pharmaceutical carrier such as lactose, starch, dextrin, sucrose, crystalline cellulose, kaolin, calcium carbonate, talc or magnesium stearate. An injection preparation is prepared by dissolving the compound in distilled water or a solution of salt such as sodium chloride or potassium chloride.

The dosage of the compounds of this invention is 5 - 300 mg/Kg/day, preferably 10 - 150 mg/Kg/day, for oral administration, and is 0.5 - 100 mg/Kg/day, preferably 1 - 50 mg/Kg/day, for parenteral administration. The desired amount is administered in a single dose or in several doses daily.

This invention is now described in greater detail by reference to the following experiment and examples to which this invention is by no means limited.

Experiment: Effect of preventing acute pulmonary thromboembolic death of mice

Six-week-old male ddY/SPF strain mice were administered orally with 200 mg/Kg of the compounds of Examples 9 and 10 and the active controls. Four hours later, the mice were intravenously administered with 325 mg/Kg of ADP and 140 mg/Kg of sodium arachidonate. The number of the mice that were dead within 24 hours of the administration of ADP and sodium arachidonate was counted and the results are shown in the table below. As a reference compound, ticlopidine and sulfinpirazone were used and their efficacy to prevent acute thrombosis was compared with that of the compounds of this invention.

Table

Effect of Compounds of the invention against Acute Pulmonary Thromboembolic Death

| Test Compound | ADP | | Arachidonic Acid | |
|---|---|---|---|---|
| | No. of Dead Mice | Inhibition% | No. of Dead Mice | Inhibition% |
| Ticlopidine | 9/20 | 40 | 7/20 | 36 |
| Sulfinpirazone | 16/20 | -7 | 8/20 | 27 |
| Compound of Ex. 9 | 9/20 | 40 | 4/20 | 64 |
| Compound of Ex. 10 | 6/20 | 60 | 7/20 | 36 |
| Control | 30/40 | - | 11/20 | - |

Example 1

Twenty-seven grams of N-nicotinoyl-2-methoxyaniline was dissolved in 800 ml of 10% hydrous ethanol. To the solution, 5 g of 10% palladium-on-carbon was added and the mixture was hydrogenated at 40 to 50°C under atmospheric pressure. After 2 mols of hydrogen was introduced over a period of about 6 hours, the reaction was stopped and the catalyst was removed. The liquid reaction mixture was concentrated and the residual oil was purified with column chromatography on silica gel and recrystallized from hydrous methanol to give N-(1,4,5,6-tetrahydronicotinoyl)-2-methoxyaniline having a melting point of 126-128°C. Yield=60%

Elemental analysis:

Calculated for $C_{13}H_{16}N_2O_2$: C 67.2; H 6.9; N 12.1 (%)

Found : C 67.2; H 6.8; N 12.0 (%)

Example 2

By reducing and purifying N-nicotinoyl-3-methoxyaniline as in Example 1, N-(1,4,5,6-tetrahydronicotinoyl)-3-methoxyaniline having a melting point of 173 to 176°C was produced. Yield=58%

Elemental analysis:

Calculated for $C_{13}H_{16}N_2O_2$: C 67.2; H 6.9; N 12.1 (%)

Found : C 67.3; H 7.0; N 12.2 (%)

Example 3

As in Example 1, N-nicotinoyl-4-methoxyaniline was treated and recrystallized from ethyl acetate/ether to give N-(1,4,5,6-tetrahydronicotinoyl)-4-methoxyaniline having a melting point of 112 to 115°C. Yield=64%

Elemental analysis:

Calculated for $C_{13}H_{16}N_2O_2$: C 67.2; H 6.9; N 12.1 (%)

Found : C 67.1; H 6.7; N 12.0 (%)

Example 4

As in Example 1, N-nicotinoyl-2,6-dimethylaniline was reduced at 45°C and the crystal formed by concentration was recrystallized from hydrous methanol to give N-(1,4,5,6-tetrahydronicotinoyl)-2,6-dimethylaniline having a melting point of 119 to 122°C. Yield=62%

Elemental analysis:

Calculated for $C_{14}H_{18}N_2O$: C 73.0; H 7.9; N 12.2 (%)

Found : C 72.9; H 7.8; N 12.1 (%)

Example 5

As in Example 1, N-nictinoyl-2-methylaniline was reduced and purified, and recrystallized from methanol/ether to give N-(1,4,5,6-tetrahydronicotinoyl)-2-methylaniline having a melting point of 141 to 144°C. Yield=61%

Elemental analysis:

Calculated for $C_{13}H_{16}N_2O$: C 72.2; H 7.5; N 13.0 (%)

Found : C 72.1; H 7.6; N 13.1 (%)

Example 6

Fifty grams of N-nicotinoyl-2-fluoroaniline was dissolved in 700 ml of 10% hydrous ethanol. To the solution, 5 g of 10% palladium-on-carbon was added and the mixture was reduced with hydrogen at room temperature and at atmospheric pressure. When a stoichiometric amount of hydrogen was absorbed, the reaction was stopped and the catalyst was removed. The liquid reaction mixture was concentrated and the resulting crystal was recrystallized from hydrous methanol to give N-(1,4,5,6-tetrahydronicotinoyl)-2-fluoroaniline having a melting point of 137 to 140°C. Yield=70%

Elemental analysis:

Calculated for $C_{12}H_{13}N_2OF$: C 65.4; H 5.9; N 12.7 (%)

Found : C 65.2; H 5.7; N 12.5 (%)

Example 7

By reducing N-nicotinoylaniline as in Example 6 except that the hydrogen pressure was 4 atm., N-(1,4,5,6-tetrahydronicotinoyl)aniline having a melting point of 209 to 212°C was produced. Yield=63%

Elemental analysis:

Calculated for $C_{12}H_{14}N_2O$: C 71.3; H 7.0; N 14.0 (%)

Found : C 71.4; H 7.1; N 13.8 (%)

Example 8

As in Example 6, 2-nicotinamidopyridine was reduced and recrystallized from tetrahydrofuran ether to give 2-(1,4,5,6-tetrahydronicotinamido)pyridine having a melting point of 156 to 159°C. Yield=68%

Elemental analysis:

Calculated for $C_{11}H_{13}N_3O$: C 65.0; H 6.5; N 20.7 (%)

Found : C 64.9; H 6.4; N 20.6 (%)

Example 9

As in Example 1, N-nicotinoyl-2,5-dimethoxyaniline was hydrogenated. After removing the catalyst, the solvent was concentrated and the resulting oil was purified by column chromatography on silica gel and recrystallized from methanol/ether to give N-(1,4,5,6-tetrahydronicotinoyl)-2,5-dimethoxyaniline having a melting point of 124 to 126°C. Yield=60%

Elemental analysis:

Calculated for $C_{14}H_{18}N_2O_3$: C 64.1; H 6.9; N 10.7 (%)

Found : C 64.0; H 6.8; N 10.8 (%)

Example 10

As in Example 1, N-nicotinoyl-2-ethoxyaniline was hydrogenated. After removal of the catalyst, the solvent was concentrated and the resulting crystal was recrystallized from methanol/water to give N-(1,4,5,6-tetrahydronicotinoyl)-2-ethoxyaniline having a melting point of 145 to 146°C. Yield=62%

Elemental analysis:

Calculated for $C_{14}H_{18}N_2O_2$: C 68.3; H 7.4, N 11.4 (%)

Found : C 68.2; H 7.5; N 11.3 (%)

We claim:

1. A 1,4,5,6-tetrahydronicotinamide derivative of the formula:

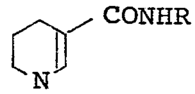

(wherein R is a phenyl or pyridyl group which may have one or more substituents) or a salt thereof.

2. A 1,4,5,6-tetrahydronicotinamide derivative of the formula:

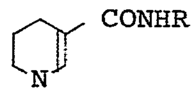

(wherein R is a phenyl or pyridyl group which may have one or more alkyl groups containing 1-4 carbon atoms, alkoxy groups containig 1-4 carbon atoms or halogen atoms as substituents) or a salt thereof.

3. N-(1,4,5,6-Tetrahydronicotinoyl)-2-methoxyaniline.

4. N-(1,4,5,6-Tetrahydronicotinoyl)-3-methoxyaniline.

5. N-(1,4,5,6-Tetrahydronicotinoyl)-4-methoxyaniline.

6. N-(1,4,5,6-Tetrahydronicotinoyl)-2,5-dimethoxyaniline.

7. N-(1,4,5,6-Tetrahydronicotinoyl)-2-ethoxyaniline.

8. N-(1,4,5,6-Tetrahydronicotinoyl)-2-methylaniline.

9. N-(1,4,5,6-Tetrahydronicotinoyl)-2,6-dimethylaniline.

10. N-(1,4,5,6-Tetrahydronicotinoyl)-2-fluoroaniline.

11. N-(1,4,5,6-Tetrahydronicotinoyl)aniline.

12. 2-(1,4,5,6-Tetrahydronicotinamido)pyridine.

13. A process for producing a 1,4,5,6-tetrahydronicotinamide derivative of the formula:

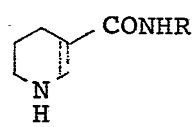

(wherein R is a phenyl or pyridyl group which may have one or more substituents) by partially reducing a nicotinamide derivative of the formula:

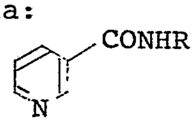

(wherein R is the same as defined above).

14. A process according to Claim 13 wherein the reaction is performed by introducing hydrogen gas in the presence of a catalyst.

15. A process according to Claim 14 wherein the catalyst is palladium-on-carbon or Raney-nickel.

16. A process according to Claim 14 wherein the reaction is performed at a temperature between 0 and 100°C.

17. A process according to Claim 16 wherein the temperature is between 10 and 50°C.

18. A process according to Claim 14 wherein the reaction is performed at a hydrogen pressure between 1 and 50 atm.

19. A process according to Claim 18 wherein the hydrogen pressure is between 2 and 5 atm.

20. A process according to Claim 14 wherein the reaction is performed in a solvent selected from the group consisting of tetrahydrofuran, dioxane, alcohol, hydrous alcohol and acetic acid.

21. A pharmaceutical composition comprising a 1,4,5,6-tetrahydronicotinamide derivative of the formula:

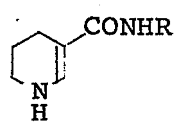

(wherein R is a phenyl or pyridyl group which may have one or more substituents) or a slat thereof and a pharmaceutically acceptable carrier.

22. A pharmaceutical composition according to Claim 21 which is formulated in the form of a tablet, granule, powder or capsule for oral administration.

23. A pharmaceutical composition according to Claim 22 which uses lactose, starch, dextrin, sucrose, crystalline cellulose, kaolin, calcium carbonate, talc or magnesium stearate as a carrier.

24. A pharmaceutical composition according to Claim 24 which enables the administration of the tetrahydronicotinamide derivative in an effective dose of 5 - 300 mg/Kg per day.

25. A pharmaceutical composition according to Claim 24 which enables the administration of the tetrahydronicotinamide derivative in an effective dose of 10 - 150 mg/Kg per day.

26. A pharmaceutical composition according to Claim 21 which is formulated in the form of an injection.

27. A pharmaceutical composition according to Claim 26 which uses distilled water or an aqueous solution of sodium chloride or potassium chloride as a carrier.

28. A pharmaceutical composition according to Claim 26 which enables the administration of the tetrahydronicotinamide derivative in an effective dose of 0.5 - 100 mg/Kg per day.

29. A pharmaceutical composition according to Claim 26 which enables the administration of the tetrahydronicotinamide derivative in an effective dose of 1 - 50 mg/Kg per day.

30. A pharmaceutical composition according to Claim 21 which is an agent to prevent and cure thrombosis.

31. A pharmaceutical composition according to Claim 21 which is an antiinflammatory agent.

32. A pharmaceutical composition according to Claim 21 which is an antipyretic analgesic agent.

0083378

European Patent Office

# EUROPEAN SEARCH REPORT

Application number

EP 81 11 0691

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | JOURNAL OF ORGANIC CHEMISTRY, vol. 31, no. 8, published in August 1966 in Easton, USA, pages 2487-2490 P.M. QUAN et al.: "1,4,5,6-tetrahydropyridines from catalytic reduction of nicotinoyl derivatives and their ring opening with hydrazine" * Whole article * --- | 1,13-18,20 | C 07 D 211/78<br>C 07 D 401/12<br>A 61 K 31/455 |
| A | EP-A-0 000 167 (KROGSGAARD-LARSEN P.) --- | 1,21-25 | |
| A | EP-A-0 011 447 (I.C.I.) --- | 1,21-25 | |
| A | TETRAHEDRON, vol. 29, 1973, Pergamon Press, pages 3991-3993, G.B. U. KRAATZ: "Zur Addition von Arylisocyanaten an gamma-Butyrolactim- und delta-Valerolactimäther" ----- | 1,2 | TECHNICAL FIELDS SEARCHED (Int. Cl. 3)<br>C 07 D 211/00<br>C 07 D 401/00<br>A 61 K 31/00 |

The present search report has been drawn up for all claims

| Place of search | Date of completion of the search | Examiner |
|---|---|---|
| THE HAGUE | 18-03-1982 | NUYTS A.M.K.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82